Europäisches Patentamt

European Patent Office

Office européen des brevets

(11) Publication number: **0 289 129**
**A2**

## EUROPEAN PATENT APPLICATION

(21) Application number: 88302562.9

(22) Date of filing: 23.03.88

(51) Int. Cl.⁴: **C12P 21/02 , C07K 3/12**

The applicant has filed a statement in accordance with Rule 28 (4) EPC (issue of a sample only to an expert). Accession number of the deposit: NRRL B - 15910.

(30) Priority: 26.03.87 US 30934
06.08.87 US 83544

(43) Date of publication of application:
02.11.88 Bulletin 88/44

(84) Designated Contracting States:
AT BE CH DE ES FR GB GR IT LI LU NL SE

(71) Applicant: **REPLIGEN CORPORATION**
**101 Binney Street**
**Cambridge Massachusetts 02142(US)**

(72) Inventor: **Love, Richard N.**
**50 Winsor Avenue**
**Watertown Massachusetts 02172(US)**
Inventor: **Profy, Albert T**
**28 Essex Street**
**Cambridge Massachusetts 02139(US)**

(74) Representative: **Perry, Robert Edward et al**
**GILL JENNINGS & EVERY 53-64 Chancery Lane**
**London WC2A 1HN(GB)**

(54) High purity protein A preparation.

(57) A process for reducing the protein contamination level of a protein A preparation, comprises heating the preparation at 50 to 100°C for 10 to 60 min.

A process for preparing substantially pure protein A comprises the steps of:

(a) heating a protein A-containing solution or other protein A preparation at 50 to 100°C for 10 to 60 min;

(b) passing the re-dissolved protein A-containing solution over an ion-exchange column;

(c) eluting protein A from the ion-exchange column: and

(d) precipitating the eluted protein A with ethanol.

EP 0 289 129 A2

# HIGH PURITY PROTEIN A PREPARATION

## Background of the Invention

Downstream processing of expressed recombinant proteins is an important consideration in the utilization of cloned gene products. Specifically, purification at industrial levels must supply a protein of sufficient purity in a cost effective manner.

Protein A is a well-known, useful protein originally derived from the cell wall of Staphylococcus aureus. It has the ability to bind the Fc region of most mammalian antibodies of class IgG. Commercial applications of protein A utilizing this property include large-scale affinity purification of monoclonal antibodies, many of which will have therapeutic applications; therapeutic plasma exchange, in which the circulating immune complexes from blood plasma are removed via binding to immobilized protein A; as well as numerous laboratory applications based on immunochemical or histochemical procedures.

To meet these commercial demands, protein A must be economically produced in large quantities and, for therapeutic applications, the protein A must be greater than 99% pure as determined by a variety of assay methods including SDS polyacrylamide gel electrophoresis (PAGE) and size-exclusion HPLC. In addition, contaminating endotoxin levels, especially important in protein A preparations produced from Gram-negative hosts, must be very low, on the order of <1.0 endotoxin units (E.U.)/mg protein A as determined by the gel clot assay (LAL).

These are currently two published methods used to purify protein A. One method uses an IgG affinity column, and the other is a multistep, small scale process which includes the use of a diethylaminoethyl (DEAE) ion-exchange column followed by the use of a sizing column.

The first method utilizes the binding of protein A to the Fc region of the IgG molecule. As developed by Hjelm et al. (Hjelm, H., Hjelm, K., Sjoquist, J [1972] FEBS Letters 28:73-76), IgG is bound to an insoluble matrix which is then packed into a column. A protein A-containing solution is passed over the column at neutral pH in 0.1 M $H_2PO_4^-$. Under these conditions protein A is selectively removed from the solution due to its interaction with the immobilized IgG. The protein A is eluted from the column with 0.1 M glycine-HCl, pH 3.0 and comes off with the buffer front.

Protein A also has been purified using a multistep procedure (Sjoquist, J., Meloun, B., Hjelm, H., [1972] Eur. J. Biochem. 29:572-578). S. aureus, strain Cowan I (NCTC 8530, National Collection of Type Cultures, Central Public Health Laboratory, London, England) is pelleted and resuspended in 0.05 M Tris-HCl [Tris = Tris(hydroxymethyl)aminomethane], 0.145 M NaCl pH 7.5 and heated to 37°C. Lysostaphin and DNAase are added and digestion allowed to go to completion as monitored by absorbance at 520 nm. After the absorbance values become constant, the suspension is centrifuged and the supernatant is treated with 5 N HCl to lower its pH to 3.5. The resulting precipitate is removed by centrifugation and the supernatant adjusted to pH 7.0 with 5 N NaOH. Ammonium sulfate is added to make an 80% saturated solution. This material is centrifuged and the precipitate removed, redissolved in 0.1 M ammonium bicarbonate, pH 8.0, and applied to a DEAE-SEPHADEX® (SEPHADEX is a tradename of Pharmacia Inc., Piscataway, N.J.) column equilibrated in 0.1 M ammonium bicarbonate, pH 8.0. Under these conditions, the protein A molecule will bind to the positively charged DEAE groups on the column. Protein A is then eluted by increasing the ionic strength of the column buffer via a linear gradient to 0.4 M ammonium bicarbonate, pH 8.0.

The protein A removed from the column is concentrated and applied to a SEPHADEX®G-100 column equilibrated in 0.05 M Tris•HCl, 1.0 M NaCl pH 8.0. On such a column proteins are separated according to size--larger molecules are excluded from the interior of the porous SEPHADEX® and therefore pass through the column more rapidly than smaller molecules which can enter the column packing.

While these purification schemes give satisfactory results for laboratory use, they are inadequate for industrial-scale applications. The problems associated with the first procedure include the high cost of preparation and maintaining an immobilized IgG column. The IgG must be isolated before any work toward purifying protein A can begin. Also, the immobilization of IgG may involve the use of a highly toxic material, cyanogen bromide. Another problem occurs because a biological molecule is part of the column packing. This means that much care must be taken to prevent its contamination or degradation and concomitant loss of binding activity. In spite of such measures, column life is considerably less than that of other properly maintained column packings.

The second purification scheme, discussed above, also is limited for large-scale work due to several reasons. This is a multistep procedure involving acid precipitation, ammonium sulfate precipitation, and buffer exchange before reaching the ion-exchange and sizing column steps. Because each step of a purification results in some loss of material, the overall number of steps should be kept to a minimum. Another drawback to this method is the amount of ammonium bicarbonate used during the ion-exchange chromatography. The cost of using a gradient from 100 mM to 400mM ammonium bicarbonate would be a substantial portion of the purification cost. The most serious drawback of the procedure, however, is the use of a sizing column. While such columns are suitable for laboratory operations, use of a sizing column at industrial levels is undesirable because capacity is very low (sample size is limited to 2-5% of the column volume),processing time is quite slow compared to other chromatographic methods, and, finally, sizing columns are often troublesome sources of contaminating endotoxins due to bacterial growth in the column.

Finally, both procedures, discussed above, involve the use of pH 3.0-3.5 solutions. This is not suitable for use in the purification of recombinant protein A expressed in E. coli as these organisms contain proteases which will degrade protein A at low pH's.

Thus, there is a need in the art for an efficient process to purify protein A-containing solutions on an industrial scale to obtain high-purity protein A preparations with acceptable low levels of endotoxin. The need is particularly great in the recombinant DNA art of producing protein A, as shown above.

## Brief Summary of the Invention

The subject invention concerns an efficient large-scale process for purifying a protein A-containing solution or other protein A preparation. The final yield, advantageously, is extremely high and purity of the final product is greater than 99%. In addition, the final product is suitable for therapeutic applications because of its extremely low endotoxin levels, <1.0 E.U./mg protein A. More specifically, the subject invention process utilizes a heat step in which the majority of the contaminating proteins are precipitated.

The resulting clarified protein A-containing solution is applied to an ion-exchange material which is packed into a column. This material can be an anion-exchange or a cation-exchange matrix. The anion-exchange matrix can consist of primary, secondary, or quaternary amino groups, e.g., aminoethyl, diethylaminoethyl, and diethyl(2-hydroxypropyl)aminoethyl groups, bound to a solid support. The cation-exchange matrix can consist of a negatively charged group, such as carboxymethyl or sulfopropyl groups, bound to a solid support. The solid support may be any insoluble, hydrophilic substance, e.g. cellulose, agarose, TRISACRYL® (Reg. Trademark, Reactifs IBF--Société Chimique Pointet-Girard, Villeneuve-La-Garenne, France), or crosslinked dextran. Anion-exchange materials are available under the trade names DEAE-SEPHAROSE® FAST FLOW, Q SEPHAROSE® FAST FLOW, and DEAE-SEPHACEL® (Reg. Trademarks, Pharmacia, Inc., Piscataway, NJ). Cation-exchange materials are available under the trade names CM-SEPHAROSE® FAST FLOW, S SEPHAROSE® FAST FLOW, and CM-SEPHACEL® (Pharmacia).

Upon washing with a buffer containing a non-ionic detergent followed by the application of an elution buffer with increased ionic strength or changed pH, this column yields a protein A solution which is typically in excess of 95% purity, with endotoxin levels attainable to below 1.0 E.U./mg protein A. The protein A is then precipitated by the addition of ethanol. Contaminating proteins are removed with the supernatant.

## Detailed Disclosure of the Invention

The invention process comprises:
1. heating a protein A-containing solution at a temperature of about 50°C to about 100°C for about 10 min to about 60 min;
2. passing the redissolved protein A-containing solution over an ion-exchange column;
3. eluting protein A from the ion-exchange column; and
4. precipitating the eluted protein A with ethanol to give a substantially pure preparation of protein A.

Optionally, a precipitation step can be added after step 1. In this step the protein A can be precipitated with ethanol. The resulting protein A pellet can be dissolved in a buffer.

A washing step can be used, advantageously, after step 3 above, to remove contaminating proteins which are not removed in the heating step. As disclosed above, the majority of contaminating proteins are removed by the heating step. Thus, the heating step is critical.

The range of temperature and time for the heating step can be varied to give the best balance of purity and yield of protein A. This adjustment is well within the skill of a person in the art and does not require undue experimentation.

Where the starting protein A-containing solution is obtained from a recombinant source, e.g., a protein A E. coli culture, then the protein A preparation obtained by the invention process, advantageously, has a low level of endotoxin.

Since the greatest utility for the subject invention process will be in recombinant protein A recovery systems, the invention process is specifically exemplified hereinafter by reference to such a system.

Several features of this process make it quite suitable for industrial-scale production of therapeutic-quality protein A. Process time is kept to a minimum as each step in the process is a batch step with corresponding advantages in throughput. Another advantage of the batch nature of each of the steps is their large capacity--each is limited primarily by the volume of the contianers or columns employed. The high throughput and large capacity of this process is in sharp contrast with the limitations of any process utilizing sizing columns. In addition, the column matrix used in the ion-exchange portion of this process is very stable, resulting in low maintenance costs as well as limiting the number of times it must be replaced. This avoids those problems typically associated with the use of bound IgG in affinity chromatography.

The reduction in endotoxin levels to the very low levels necessary for therapeutic applications is possible because of the buffer composition used in the ion-exchange column. The use of a non-ionic detergent allows the disassociation of protein A and endotoxin resulting in a reduction to less than 1.0 E.U./mg protein A, as opposed to the >10 E.U./mg protein A levels which are obtained when no detergent is used in the process. An affinity chromatography column can be used in place of the ion-exchange column.

Finally, the use of ethanol precipitation in this process combines purification, buffer exchange, and concentration in a single step. Such efficiency is critical to industrial scale processes.

The Materials and Methods used in this disclosure of the invention process are as follows:

## (1) Preparation of protein A via recombinant host microbe

Recombinant host E. coli NRRL B-15910 was grown under suitable conditions to prepare protein A. Fermentation was performed in a 500-L ABEC fermenter (ABEC, Inc. Bethlehem, PA) fitted with $dO_2$ and pH control. Recombinant cells were grown at $dO_2$ of greater than 50% (air = 100%) of saturation at the pH indicated. pH was adjusted by the addition of 85% lactic acid (U.S. Biochemical, Cleveland, OH). Foam was controlled by the addition of antifoam B (Sigma Chemical, St. Louis, MO). Fermentation temperature was 34°C. All fermentations were conducted with a final volume of 350 L in modified 2% medium.

Modified 2% medium:
2.0 g/L potassium phosphate, monobasic (Mallinckrodt Inc., St. Louis, MO)
2.0 g/L potassium phosphate, dibasic (Mallinckrodt Inc.)
2.0 g/L sodium phosphate, dibasic, heptahydrate (Mallinckrodt Inc.)
20.0 g/L casein peptone (Marcor Development Corp., Hackensack, NJ)
20.0 g/L Casamino acids (Marcor Development Corp.)
20.0 g/L yeast extract (Marcor Development Corp.)
20 mg/L chloramphenicol (U.S. Biochemical)
pH adjusted to 6.8 with 0.1 N NaOH

## (2) Buffers for anion-exchange chromatography

Any biologically compatible buffer between about pH 6 and about pH 10 can be used in the subject invention. These buffers may or may not contain substances suitable for inhibiting proteases or stabilizing protein products against oxidation. In addition, the presence of a non-ionic detergent is necessary in the specified column equilibration and wash buffers. Below are buffers which can be used.

Anion-Exchange Column Equilibration Buffer #1[*]

0.25 M Tris-HCl pH 8.3
2 mM potassium ethylenediamine-
    tetraacetic acid (KEDTA)
0.1 mM phenylmethylsulfonyl-
    fluoride (PMSF)
0.2% TRITON® X-100

Anion-Exchange Column Equilibration Buffer #2[*]

0.25 M Tris-HCl pH 8.3
2 mM KEDTA
0.1 mM PMSF
0.2% TRITON® X-100

Anion-Exchange Column Wash Buffer #1[*]

Same as Anion-Exchange
Column Equilibration Buffer #2

Anion-Exchange Column Wash Buffer #2[*]

25 mM Tris-HCl pH 8.3

Anion-Exchange Column Elution Buffer[*]

25 mM Tris-HCl pH 8.3
150 mM KCl

(3) Buffers for cation-exchange chromatography

Any biologically compatible buffer between about pH 3 and pH 6 can be used for the subject invention These buffers may or may not contain substances suitable for inhibiting proteases or stabilizing protein products against oxidation. In addition, the presence of a nonionic detergent is necessary in the specified column equilibration and wash buffers. Below are buffers which can be used.

| | |
|---|---|
| Cation-Exchange Column Equilibration Buffer #1* | 0.5 M sodium acetate (pH 4.5) 1% TRITON® X-100 |
| Cation-Exchange Column Equilibration Buffer #2* | 50 mM sodium acetate (pH 4.5) 1% TRITON® X-100 |
| Cation-Exchange Column Wash Buffer #1* | Same as Cation-Exchange Column Equilibration Buffer #2 |
| Cation-Exchange Column Wash Buffer #2* | 50 mM sodium acetate (pH 4.5) |
| Cation-Exchange Column Elution Buffer #1* | 50 mM sodium acetate (pH 4.5) 0.1 N KCl |
| Cation-Exchange Column Elution Buffer #2* | 50 mM sodium acetate (pH 4.5) 0.3 N KCl |

*Buffers are prepared pyrogen-free and are filtered through a 0.20-nm depyrogenated filter prior to use.

(4) Analytical size-exclusion HPLC

A 7.5 x 300 mm LKB TSK G3000 SW column (LKB) is equilibrated in 100 mM potassium phosphate pH 6.8. A chart speed of 0.5 cm/min is used with a flow rate of 0.5 ml/min. A 15-$\mu$g peak will be 80% full scale at 2.0 AUFS (Absorption Units Full Scale, 214 nm).

(5) Gel electrophoresis

A 1 mg/ml solution of protein A is made by the addition of 1 ml of the 10 mg/ml test solution to 9 ml of water. 100 $\mu$l of this solution is added to 100 $\mu$l of a solution containing 50% glycerol, 5% sodium dodecyl sulfate, 5% 2-mercaptoethanol and a trace of bromphenol blue. This mixture is heated to 100°C for 10 min, then cooled to room temperature. 10 $\mu$l of this solution (5 $\mu$g protein A) are electrophoresced on a 12% acrylamide SDS gel prepared according to the method of Laemmli (Laemmli, U.K. [1970] Nature 227: 680-685). The separating gel contains 9.6 ml of acrylamide solution (37.5% acrylamide, 1% bisacrylamide), 7.5 ml of 1.5 M Tris-HCl pH 8.3 buffer, 0.15 ml of 20% SDS, 9.6 ml of degased water, 0.75 ml of 1.5% ammonium persulfate, and 2.4 ml of 0.5 tetramethylethylenediamine (TEMED). The stacking gel contains 0.96 ml acrylamide solution, 2.5 ml of 0.5 M Tris-HCl pH 6.8, 0.05 ml 20% SDS, 5.0 ml of degased water, 0.7 ml 1.5% ammonium persulfate, and 0.8 ml of 0.5% TEMED.

## (6) Limulus Amebocyte Lysate (LAL) assay

Endotoxin levels of purified protein A were determined using the procedure recommended by the manufacturer (Associates of Cape Cod, Inc., Woods Hole, MA).

## (7) Sterility

The absence of microbial contamination in the purified protein A was demonstrated by plating 100 $\mu$l of a 10 mg/ml test solution of purified protein A on blood agar and YT plates (8 g tryptone, 5 g yeast extract, and 5 g NaCl/L). The plates were incubated for 48 hr at 37°C.

Following are examples which illustrate procedures, including the best mode, for practicing the invention. These examples should not be construed as limiting. All percentages are by weight and all solvent mixture proportions are by volume unless otherwise noted.

## Example 1--Lysis

A 12-kg wcw (wet cell weight) cell pellet of E. coli NRRL B-15910, containing a plasmid coding for the production of protein A, is resuspended in homogenization buffer (e.g., 8.75 mM Tris-HCl, pH 8.3, containing 2 mM KEDTA and 0.6 M PMSF). The cell suspension is passed through a Dyno-Mill® KDL-15 Model (Dyno-Mill® [a bead mill] supplied by Impandex, Maywood, NY). The Dyno-Mill® is charged with 10 L of dry glass beads (0.5-0.7 mm diameter) and operated at the highest speed setting. The cell suspension is fed to the Dyno-Mill® at a rate of 1200 ml/min. After the cell suspension has passed through the bead mill, the mill is washed with an additional 20 L of homogenization buffer.

The lysate is clarified by passage through a 0.20-$\mu$m Microgen filter (Microgon, Laguna Hills, CA). The retained volume is taken to 40% of the starting volume and washed with 3 volumes of additional homogenization buffer. The pH of the filtrate is adjusted to about 8.3 with 1 N NaOH. The pH range can be about 8.2 to about 8.4.

## Example 2--Heat step

The pH 8.3 clarified lysate, obtained in Example 1, is pumped into a jacketed steam-heated vessel (ABEC) and heated to about 80°C for about 30 min. The resulting precipitate is allowed to settle and the supernatant decanted. The precipitate contains only contaminating proteins and is discarded.

The protein A-containing supernatant is clarified by passage through a 0.2 $\mu$m Microgon filter as described in Example 1. The pH of the filtrate is adjusted to about 8.3 with 1 N NaOH and the conductivity adjusted to 4.5 mS/cm by diluting with distilled water.

## Example 3--First ethanol precipitation step (optional)

The filtrate from the heat precipitation step is passed through a 10,000 NMWL spiral filter, e.g., Amicon S 10Y30, Amicon Corp., Danvers, MA, until the retentate volume is reduced to 10 L. Ethanol is added to the retentate to a final concentration of 70%, and the protein A is allowed to precipitate. The supernatant is decanted and the pellet resuspended in 100 L of a suitable buffer (e.g., 25 mM Tris-HCl, pH 8.3, containing 2 mM KEDTA and 0.1 mM PMSF)

7

## Example 4--Anion-exchange column

The resuspended pellet, obtained in Example 3, is loaded onto a 63 x 15 cm column packed with DEAE-SEPHAROSE® FAST FLOW (Pharmacia). A 0.20-$\mu$m filter is connected to the inlet port of this column to prevent microbial contamination. Prior to loading, the packed, depyrogenated column (depyrogenated according to packing manufacturer's instructions) is equilibrated by pumping 200 L of Anion-Exchange Column Equilibration Buffer #1 through the column at a flow rate of 8 L/min. (This flow rate is maintained through all ion-exchange column operations.) 200 L of Anion-Exchange Column Equilibration Buffer #2 are then pumped through the column. The column is then loaded with the pH 8.3 filtrate. The column is washed with 400 L of Anion-Exchange Column Wash Buffer #1, followed by 400 L of Anion-Exchange Column Wash Buffer #2. Finally, the column is eluted with 100 L of Anion-Exchange Column Elution Buffer. The eluted peak is collected and assayed for protein A using size-exclusion HPLC and for endotoxin using the LAL assay. It is found that most of the contaminating proteins remaining after the heat step are removed by the column wash step. In additon, a 10-fold decrease in endotoxin levels is routinely obtained. This step may be repeated to obtain endotoxin levels as low as necessary.

## Example 5--Cation-exchange column

The pH of the resuspended ethanol pellet from Example 3 is adjusted to 4.5 and the conductivity to 3.5. It is then loaded on a 63 x 15 cm column packed with CM-SEPHAROSE® FAST FLOW. A 0.20-$\mu$m filter is connected to the inlet port of this column to prevent microbial contamination. Prior to loading, the packed, depyrogenated column (depyroginated according to the manufacturer's instructions) is equilibrated by pumping 200 L of Cation-Exchange Column Equilibration Buffer #1 through the column at a flow rate of 8 L/min. (This flow rate is maintained through all column operations.) 200 L of Cation-Exchange Column Equilibration Buffer #2 are then pumped through the column. The column is then loaded with the resuspended ethanol pellet. The column is next washed with 400 L of Cation-Exchange Column Wash Buffer #1 and then with 400 L of Cation-Exchange Column Wash Buffer #2. Next, the column is washed with 100 L of Cation-Exchange Column Elution Buffer #1. Finally, the column is eluted with Cation-Exchange Column Elution Buffer #2. The eluted peak is collected, neutralized, and assayed for protein A using size-exclusion HPLC and for endotoxin using the LAL assay. Final endotoxin levels are routinely less than 1 E.U./mg.

## Example 6--Ethanol precipitation

The eluted protein A from Examples 4 or 5, at the desired endotoxin level, is then precipitated by dilution with 3 parts ethanol to 1 part eluate. The protein A precipitate is allowed to settle and the ethanol supernatant is discarded. The 99% pure protein A pellet is resuspended in the buffer of choice.

## Example 7--Reducing endotoxin levels in other protein preparations

The above examples use a protein A preparation as the starting material. Endotoxin levels in other protein preparations also can be reduced by following essentially the same procedure, to wit:

(a) binding of the protein in the protein preparation to an immobilized support,

(b) washing the immobilized support containing the bound protein with a non-ionic detergent, and

(c) eluting the protein from the immobilized support with a pyrogen-free biologically compatible buffer solution to obtain a protein preparation with a low level of endotoxin.

Any protein preparation can be processed in the above manner.

The removal of contaminating proteins from a protein A-containing solution by heating said solution to a temperature of about 50°C to about 100°C for about 10 min to about 60 min is effective when the protein A is derived from either a recombinant organism or from Staphylococcus aureus.

**Claims**

1. A process for reducing the protein contamination level of a protein A preparation, which comprises heating the preparation at 50 to 100°C for 10 to 60 min.

2. A process for preparing substantially pure protein A, which comprises the steps of:

(a) heating a protein A-containing solution or other protein A preparation at 50 to 100°C for 10 to 60 min;

(b) passing the re-dissolved protein A-containing solution over an ion-exchange column;

(c) eluting protein A from the ion-exchange column; and

(d) precipitating the eluted protein A with ethanol.

3. A process according to claim 2, wherein the ion-exchange column is an anion-exchange column which is washed with a biologically compatible buffer solution having a pH between 6 and 10.

4. A process according to claim 2, wherein the ion-exchange column is a cation-exchange column which is washed with a biologically compatible buffer solution having a pH between 3 and 6.

5. A process according to any of claims 2 to 4, wherein the ion-exchange column comprises primary, secondary, tertiary or quaternary amine functionalities.

6. A process according to claim 5, wherein the functionalities are selected from diethylaminoethyl, aminoethyl and quaternary amino, on an insoluble hydrophilic support.

7. A process according to claim 6, wherein the support is cellulose, agarose or cross-linked dextran.

8. A process according to any of claims 2 to 7, which comprises the additional step of precipitating the protein A obtained in step (a) with ethanol and re-dissolving the pellet in a buffer.

9. A process according to any preceding claim, which comprises the additional prior step of obtaining the protein A preparation from Staphylococcus aureus.

10. A process for preparing a protein preparation having a low level of endotoxin, which comprises the steps of:

(a) binding the protein in the protein preparation to an immolibilized support;

(b) washing the immobilized support-bound protein with a non-ionic detergent, and

(c) eluting the protein from the immobilized support with a pyrogen-free biologically compatible buffer solution.

11. A process according to claim 10, wherein the protein preparation is a protein A-containing solution or other protein A preparation.

12. A process according to any of claims 1 to 8 and 11, which additionally comprises the prior step of obtaining the protein A-containing solution or other protein A preparation by expression from a recombinant DNA system.

13. A process according to claim 12, wherein the recombinant DNA system comprises a recombinant protein A-producing microorganism or a prokaryotic host comprising an extrachromosomal element encoding protein A.

14. A process according to claim 13, wherein the microorganism is a Gram-negative bacterium.

15. A process according to claim 13 or claim 14, wherein the microorganism or prokaryotic host is Escherichia coli.

16. A process according to claim 15, wherein the host is E. coli NRRL B-15910.

9